# EUROPÄISCHE PATENTANMELDUNG

(11) **EP 1 872 716 A1**
(43) Veröffentlichungstag der Anmeldung: **02.01.2008**
(21) Anmeldenummer: 06013343.6
(22) Anmeldetag: 28.06.2006
(51) Int. Cl.: A61B 5/042

(54) **Telemetrisch ansteuerbare Positioniervorrichtung für Mikroelektroden**

(71) Anmelder: Deutsches Primatenzentrum GmbH, 37077 Goettingen (DE)
(72) Erfinder: Kern, Thorsten, 64342 Seeheim/Jugenheim (DE); Rörup, Hermke, 29525 Uelzen (DE); Tammer, Roland, Dr., 37075 Göttingen (DE)
(74) Vertreter: Rehberg Hüppe + Partner

(57) **Zusammenfassung**

Eine Pasitioniervorrichtung (1) weist eine Vorschubeinheit (3), die mindestens eine Mlkroelektrode (16) trägt, eine Basis (2), an der die Vorschubeinheit (3) verschieblich gelagert Ist, und einen Aktuator (4), der ansteuerbar ist, um die Vorschubeinhelt (3) gegenüber einer Basis (2) zu verschieben, auf. Der Aktuator (4) weist seinerseits einen Resonator (19) und einen elektrisch-mechanischen Wandler (20) auf, wobei zwei unterschiedliche elliptische Elgenformen des einen Resonators (18) mit dem einen Wandler (20) anregbar sind, um die Vorschubeinheit n zwei einander entgegen gesetzten Richtungen gegenüber der Basis zu verschleben.

## Beschreibung

### TECHNISCHES GEBIET DER ERFINDUNG

Die Erfindung bezieht sich auf eine Positioniervorrichtung mit einer Vorschubeinheit, die mindestens eine Mikroelektrode trägt, mit einer Basis, an der die Vorschubeinheit verschieblich gelagert ist und mit einem Aktuator, der ansteuerbar ist, um die Vorschubeinheit gegenüber der Basis zu verschieben.

Insbesondere bezieht sich die Erfindung auf eine solche Positioniervorrichtung der eingangs beschriebenen Art, mit der bei an dem Schädelknochen eines Tiers befestigter Basis eine oder mehrere Mikroelektroden innerhalb eines Bereichs des Hirns des Tiers positionierbar sind, um dort neuronale Nervenpotentiale abzuleiten.

### STAND DER TECHNIK

Aus P. Grohrock et al.: Dual-Chanel Telemetry System for Recording Vocalization-Correlated Neuronal Activity in freely moving Squirrel Monkeys", J. Neurosci Methods Vol, 76, S. 7 to 13, 1 1997 Ist eine Positioniervorrichtung bekannt, bei der die Elektroden manuell in Richtung einer Spindelachse verschoben werden, indem eine die Spindelachse aufwelsende Spindel mit einem Schraubendreher gegenüber einer Basis verdreht wird. Von an der Spindel gelagerten Mikroelektroden abgeleitete Nervenpotentiale werden über eine drahtlose Signalübortragungsstrecke an eine entfernt angeordnete Signalempfangseinheit übermittelt.

Die bekannte Positioniervorrichtung erlaubt es zwar, Nervenpotentiale bei sich frei bewegenden Tieren zu erfassen; für die Veränderung der Positionierung der Mikroelektroden längs der Spindelachse müssen die Tiere jedoch eingefangen werden. Eine Positioniervorrichtung, die eine Veränderung der Position der Mikroelektroden von einer entfernten Steuereinheit aus ermöglichen würde, wäre daher erstrebenswert.

Um die genaue Lage einer Mikroelektrode, mit der Nervenpotentiale aus dem Hirn eines Tiers abgeleitet wurden, festzustellen, ist es gängige Praxis, dass jeweilige Tier zu töten und zu sezieren. Grundsätzlich wäre es zwar auch denkbar, die Position von Mikroelektroden durch eine MRT- (magnetresonanztomographische) Untersuchung des Schädels des Tiers einschließlich der Mikroelektroden festzustellen, doch würde dies voraussetzen, dass die Lagerung der Mikroelektrode MRT-tauglich ist. Sie darf entsprechend keineriel magnetisches Material oder irgendwelche Spulen aus elektrisch leitendem Material aufweisen.

Hohe Anforderungen an jede Positioniervorrichtung für eine Mikroelektrode in dem Hirn insbesondere eines kleineren Tiers, wie beispielsweise eines Totenkopfaffen, der ais kleiner Primat Gegenstand umfangreicher wissenschaftlicher Untersuchungen Ist, bestehen vor allem im Bereich der Miniaturisierung, Die Positioniervorrichtung muss einschließlich aller am Tier anzuordnender Energieversorgung und Steuerung sehr klein und leicht sein. Gleichzeitig muss sie die Positionierung der Mikroelektroden über einen typischen. Bereich von einigen Mllllmetem (z. B. 4 mm) in feinen Schritten der Größenordnung 10 µm erlauben. Die Größe neuronaler Zellen liegt zwischen 30 und 100 µm, und das ableitbare Nervenpotential hängt stark von der Position der jeweiligen Mikroelektrode gegenüber der jeweiligen. Zelle ab.

### AUFGABE DER ERFINDUNG

Der Erfindung liegt die Aufgabe zugrunde, eine fernansteuerbare Pösitionlervorrichtung der eingangs beschriebenen Art aufzuzeigen, die das voranstehend erläuterte Anforderungsprofil erfüllt.

### LÖSUNG

Die Aufgabe der Erfindung wird durch eine Positioniervorrichtung mit den Merkmalen des unabhängigen Patentanspruchs 1 gelöst. Bevorzugte Ausführungsformen der neuen Positioniervorrichtung sind in den abhängigen Patentansprüchen 2 bis 11 beschrieben.

### BESCHREIBUNG DER ERFINDUNG

Bei der neuen Positioniervorrichtung weist der Aktuator, der ansteuerbar ist, um die Vorschubeinheit gegenüber der Basis zu verschieben, einen Resonator und einen elektrisch mechanischen Wandler auf, wobei zwei unterschledliche elliptische Eigenformen des einen Resonators mit dem einen Wandler anregbar sind, um die Vorschubeinheit in zwei einander entgegen gesetzten Richtungen gegenüber der Basis zu verschieben. Derartige Aktuatoren sind grundsätzlich bekannt. Sie werden von der Elliptec Resonant Actuator AG, Dortmund kommerziell angeboten. Besondere Vorteile dieser Aktuatoren ist, dass sie für das Verschieben der Vorschubeinheit In beiden einander entgegen gesetzten Richtungen nur einen einzigen elektrisch-mechanischen Wandler aufweisen, der mit nur einern vergleichsweise kleinen elektrischen Signal beaufschlagt werden muss, well er nicht unmittelbar zur Verschiebung der Vorschubeinheit dient, sondern eine Eigenform des Resonators anregt, der über die gesamte Zeit seiner Schwingung in der Eigenform hinweg die Vorschubeinheit verschiebt. Dies bedeutet, dass sowohl die Energieversorgung als auch ein Hochfrequenzgenerator zur Erzeugung eines elektrischen Hochfrequenzsignals für den Wandler bei der neuen Positioniervorrichtung klein und leicht gehalten werden können.

Die bekannten Aktuatoren der Elliptec Resonant Actuator AG sind zudem bereits in wesentlichen Teilen MRT-tauglich. So wird als elektrisch-mechanischer Wandler ein piezoelektrischer Wandler eingesetzt, und der Resonator besteht aus einer nicht magnetischen Legierung.

Andere Teile der kommerziell verfügbaren Aktuatoren mit dem bei der neuen Positioniervorrichtung genutzten Wirkprinzip sind jedoch als solche nicht MRT-tauglich und sollten daher für die Ausbildung der neuen Positioniervorrichtung abgeändert werden. Hierzu gehört eine Feder zum Andrücken des an der Basis abgestützten Resonators an die Vorschubeinheit. Bei den kommerziell verfügbaren Aktuatoren ist hierfür eine aus Federdraht gebogene Feder vorgesehen, die In Ihrer Mitte mehrere Windungen aus dem Federdraht aufweist. Grundsätzlich wäre diese Feder zwar beispielsweise auch durch eine Sohenkeifeder ohne Windungen aus einem nichtmagnetischen Metall ersetzbar. Dies wäre jedoch ein vergleichsweise aufwändiges Bauteil der neuen Positioniervorrichtung. Wenn stattdessen jede Feder zum Andrücken des An der Basis abgestützten Resonators an die Vorschubeinheit aus Kunststoff ausgebildet wird, bestehen grundsätzlich keinerlei Probleme bezüglich der MRT-Täuglichkeit.

Konkret kann jede Feder zum Andrücken des an der Basis abgestützten Resonators an die Vorschubeinheit ein elastisches Band sein. Dieses elastische Band kann etwa parallel zu dem Resonator verlaufen, wobei es an dem Ende des Resonators, an dem dieser verschwenkbar an der Basis gelagert ist, über einen Hebelarm an dem Resonator angreift, während es an dem Ende des Resonators, an dem dieser in der Vorschubeinheit angreift, an der Basis abgestützt ist. Die elastischen Bänder erhöhen so die gesamte Baugröße der Positioniervorrichtung nicht nennenswert. Geeignete elastische Bänder aus Kunststoff, d. h. z. B. sogenannte Gummibänder, mit ausreichender Dauerelastizität sind beispielsweise aus dem Dentalbereich bekannt und kommerziell verfügbar.

Wie bei den kommerziell verfügbaren Aktuatoren der Elliptec Resonant Actuator AG weist auch der Resonator bei der neuen Positioniervorrichtung einen typischen Winkel von 45 bis 55°, d. h. etwa 50°, zu der Vorschubeinheit auf. Die Anregung des Resonators mit dem Wandler zu den eliptischen Eigenformen führt dazu, dass dessen Ende, das seiner Abstützung an der Basis abgekehrt ist, elliptische Bewegungen vollführt, deren Drehsinn von der jeweiligen Eigenform und damit von der Frequenz der Anregung des Resonators von dem Wandler abhängt. Bei jeder elliptischen Bewegung des freien Endes des Resonators verschiebt dieser die Vorschubeinheit um einen Mikroschritt, hebt von der Vorschubeinheit ab und setzt wieder auf der Vorschubeinheit auf, wodurch die Vorschubeinheit am Ende der Schwingung des Resonators in ihrer Lage fixiert wird. Bei jeder Anregung des Resonators mit dem Wandler wird die Vorschubeinheit um eine Vleizahl solcher Mikroschritte verschoben. Bei einem definierten elektrischen Signal, mit dem der Wandler beaufschlagt wird, um den Resonator anzuregen, ergibt sich eine hinreichend definierte Gesamtverschlebung der Vorschubeinheit in der gewünschten Größenordnung von 10 µm.

Bei den kommerziell verfügbaren Aktuatoren der Firma Elliptec Resonant Actuator AG weist die Vorschubeinheit einen an der Basis über Stahlkugeln kugelgelagerten Schlitten auf, an der der Resonator angreift. Bei der neuen Positioniervorrichtung ist es aus Gewichtsgründen bevorzugt, wenn der Resonator an einer einfachen Schubstange der Vorschubeinheit angreift. Diese Schubstange kann konkret ein Rohrabschnitt aus einem mit Kohlenstofffasern verstärkten Kunststoff sein. Die Mikroelektroden können durch das Innere dieses Rohrabschnitts geführt sein.

Die Kugeln zur Kugellagerung der Schubstange bestehen bei der neuen Positioniervorrichtung vorzugsweise aus einem keramischen Material und sind damit insbesondere nicht magnetisch.

Wie bereits hervorgehoben wurde, ermöglicht die neue Positioniervorrichtung problemlos die Ansteuerung von einer entfernten Steuereinheit aus. Hierzu kann ist der Hochfrequenzgenerator zur Erzeugung des elektrischen Hochfrequenzsignals für den Wandler von der entfernten Steuereinheit über eine drahtlose Signalübertragungsstrecke ansteuerbar. Der Hochfrequenzgenerator gibt sein Hochfrequenzsignal typischerweise sinusförmig mit einer Frequenz von einigen 10 kHz aus, wobei die Frequenzen der beiden elliptischen Eigenformen des Resonators, die genutzt werden, um die Vorschubeinheit gegenüber der Basis In entgegen gesetzten Richtungen zu verschieben, um typischerweise wenige 10 kHz auseinander liegen. Die Spannung des Hochfrequenzsignals des Hochfrequenzgenerators weist eine typische Größenordnung von 5 bis 10 V auf, ist also gerade für die Ansteuerung eines piezoelektrischen Wandlers relativ niedrig.

Die drahtlose Signalübertragungsstrecke zwischen der Steuereinheit und dem Hochfrequenzgenerator kann In umgekehrter Richtung bei der neuen Positioniervorrichtung auch dazu genutzt werden, Messsignale von Jeder Mikroelektrode an die Steuereinheit zu übertragen. Es können aber auch zwei getrennte drahtlose Signalübertragungsstrecken nebeneinander vorgesehen sein.

Vorteilhafte Weiterbildungen der Erfindung ergeben sich aus den Patentansprüchen, der Beschreibung und den Zeichnungen. Die in der Beschreibungseinleitung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die In separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

### KURZBESCHREIBUNGDER FIGUREN

Im Folgenden wird die Erfindung anhand In den Figuren dargestellter bevorzugter Ausführungsbeispiele weiter erläutert und beschrieben.
- Fig.1: ist eine perspektivische Seitenansicht der neuen Positioniervorrichtung.
- Fig. 2: ist eine Explosionsansicht der Positioniervorrihtung gemäß Fig. 1, wobei deren elastische Bänder weggelassen sind.
- Fig.3: ist eine Seitenansicht der Positioniervorrichtung, wieder ohne die elastischen Bänder, wobei hier auch deren Mikroelektroden nicht wiedergegeben sind, und
- Fig. 4: skizziert eine Kugellagerung einer Schubstange der neuen Vorrichtung.

### FIGURENBESCHREIBUNG

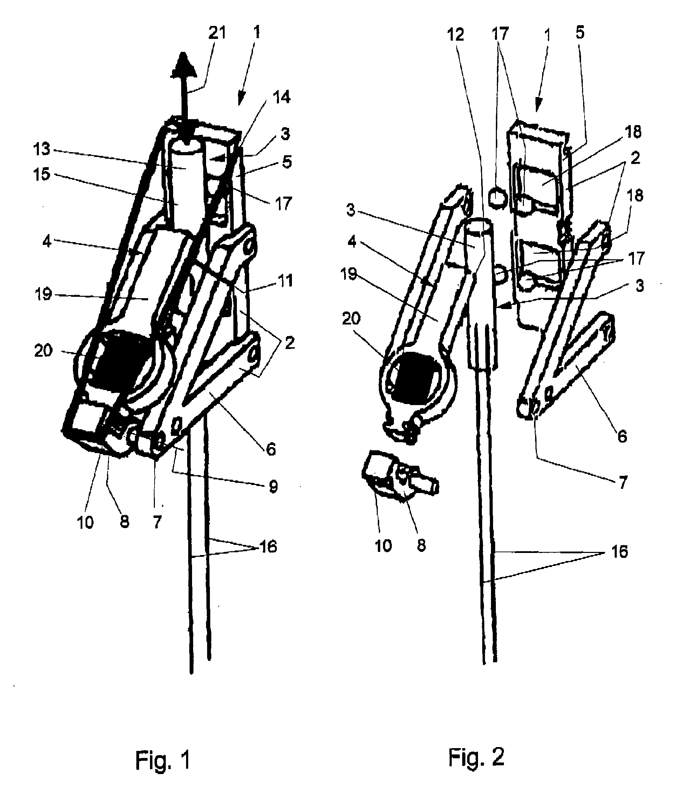

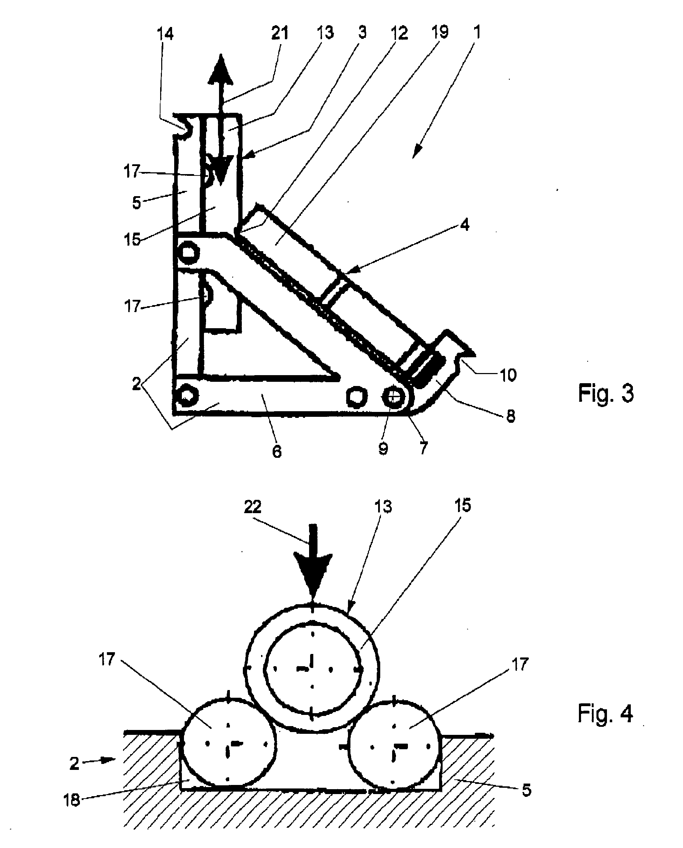

Die Positioniervorrichtung 1, die in den Fig. 1-3 dargestellt ist, wobei bestimmte Einzelteile in den Fig. 2 und 3 weggelassen sind, weist als wesentliche Bestandteile eine Basis 2, eine Vorschubeinheit 3 und einen Aktuator 4 auf. Die Basis 2 besteht aus einer Grundplatte 5, an die zwei V-förmige Elemente 6 seitlich angesetzt sind. An dem freien Ende 7 der V-förmigen Elemente 6 Ist ein Hebelarm 8 um eine Achse 9 verschwenkbar an der Basis 2 gelagert. Der Hebelarm 8 trägt den Aktuator 4 und weist weiter von der Schwenkachse 9 entfernt eine Einkerbung 10 für ein elastisches Band 11 auf. Dieses elastische Band 11 ist nur in Fig. 1 wiedergegeben. Es dient dazu, den um die Schwenkachse 9 gegenüber der Basis 2 verschwenkbar gelagerten Aktuator 4 mit seinem freien Ende 12 an eine Schubstange 13 der Vorschubeinheit 3 anzudrücken. Dabei stützt sich das elastische Band 11 in einer Einkerbung 14 In der Grundplatte 5 ab und verläuft etwa parallel zu dem Aktuator 4, auf den es über den Hebelarm 8 einwirkt. Die Schubstange 5 ist ein Rohrabschnitt 15 aus Kunststoff, der mit Kohlenstofffasern verstärkt ist, und führt im seinem Inneren zwei nach unten über die Schubstange 13 überstehende Mikroelektroden 16. Die Schubstange 13 ist über vier Kugeln 17 an der Grundplatte gelagert, wobei die Kugeln 17 in Ausnehmungen 18 in der Grundplatte 5 geführt sind. Der Aktuator 4 selbst besteht aus einem Resonator 19 und einem piezoelektrischen Wandler 20. Der piezoelektrische Wandler 20 regt bei Beaufschlagung mit einem elektrischen Signal den Resonator 19 zu mechanischen Schwingungen an. Dabei wird durch die Auswahl der Frequenz des elektrischen Signals jeweils gezielt eine von zwei elliptischen Eigenformen des Resonators 19 angeregt, bei denen das freie Ende 12 eine elliptische Bahn im Raum beschreibt. Im Verlauf dieser Bahn schiebt das freie Ende 12 die Schubstange 13 vor oder zurück, hebt von dieser ab und setzt wieder auf diese auf. Je nach der Drehrichtung der elliptischen Bahn des Endes 12 resultiert hieraus eine unterschiedliche Bewegungsrichtung der Schubstange 13. Beide möglichen Bewegungsrichtungen sind in den Fig. 1 und 3 durch einen Doppelpfell 21 angedeutet.

**Fig. 4** zeigt als Detail die Lagerung der Schubstange 13 in Form des Rohrabschnitts 15 durch die Kugeln 17 gegenüber der Grundplatte 5 der Basis 2. Die Kugeln 17 sind dabei In der Ausnehmung 18 der Grundplatte 5 geführt. Die Kugeln 17 bestehen aus nichtmagnetischem, d. h. konkret aus keramischem Material. Ein Pfeil 22 deutet die Kraft an, mit der das Ende 12 des Aktuators 4 aufgrund der Beaufschlagung mit dem elastischen Band 11 gemäß Fig. 1 an der Schubstange 13 anliegt. Diese Kraft bestimmt die Reibung zwischen dem Ende 12 und der Schubstange 13 und damit sowohl dem Vorschub der Schubstange 13 bei schwingendem Resonator 19 als auch die Haltekraft, die der Aktuator 4 im nicht schwingenden Zustand auf die Schubstange 13 ausübt.

Als elastisches Band 11 wurden bei einem konkreten Aufbau der neuen Vorrichtung 1 sieben dentale Gummibänder (Olympia Light Pull 2 ½ oz) parallel zueinander angeordnet. Der Rohrabschnitt 15 wies einen Außendurchmesser von 3 mm auf. Die Bauhöhe und die Bautiefe der Vorrichtung 1 betrugen jeweils 25 mm, die Baubrelte knapp 9 mm. Statt einer In den Figuren gezeigten Verschraubung der Teile 5 und 6 der Basis können diese auch miteinander verklebt sein, um zusätzliches Gewicht einzusparen. Selbst bei einer verschraubten Basis wog die gesamte Positioniervorrichtung einschließlich eines hier nicht gezeigten Hochfrequenzgenerators für die Ansteuerung des Wandlers 20 nur 10,5 g. Dabei wurde als Hochfrequenzgenerator die Elektronik verwendet, die von der Firma Elliptec Resonant Actuator AG mit deren Aktuatoren geliefert wird. Die beiden Frequenzen der beiden genutzten elliptischen Eigenformen des Resonators 21 lagen bei 79 kHz für einen Aufwärtsbewegung der Schubstange 13 und bei 97 kHz für eine Abwärtsbewegung der Schubstange. Eine Ansteuerung des Hochfrequenzgenerators erfolgte über eine herkömmliche Infrarotfernbedienung. Die gemessene Schrittweite bei einer einmaligen Anregung des Resonators 19 mit dem Wandler 20 lag bei 7 bis 11 µm. Die Streuung der Schrittweite wies eine statistische Verteilung auf. So mittelt sich die Streuung bei der Schrittweite der einzelnen Schritte über größere Schrittzahlen tendenziell heraus.

### BEZUGSZEICHENLISTE

- 1: Positioniervorrichtung
- 2: Basis
- 3: Vorschubeinheit
- 4: Aktuator
- 5: Grundplatte
- 8: V-förmiges Element
- 7: freies Ende
- 8: Hebelarm
- 9: Schwenkachse
- 10: Einkerbung
- 11: elastisches Band
- 12: Ende
- 13: Schubstange
- 14: Einkerbung
- 15: Rohrabschnitt
- 16: Mikroelektrode
- 17: Kugel
- 18: Ausnehmung
- 19: Resonator
- 20: Wandler
- 21: Doppelpfeil
- 22: Pfeil

## Patentansprüche

1. Positioniervorrichtung mit einer Vorschubeinheit, die mindestens eine Mikroelektrode trägt, mit einer Basis, an der die Vorschubeinheit verschieblich gelagert Ist, und mit einem Aktuator, der ansteuerbar ist, um die Vorschubeinheit gegenüber der Basis zu verschieben, **dadurch gekennzeichnet, dass** der Aktuator (4) einen Resonator (19) und einen elektrlschmechanischen Wandler (20) aufweist, wobei zwei unterschiedliche elliptische Eigenformen des einen Resonators (19) mit dem einen Wandler (20) anregbar sind, um die Vorschubeinheit (3) In zwei einander entgegen gesetzten Richtungen gegenüber der Basis (2) zu verschieben.

2. Positioniervorrichtung nach Anspruch 1, **dadurch gekennzeichnet, dass** der Wandler (20) ein piezoelektrischer Wandler ist.

3. Positioniervorrichtung nach Anspruch 1 oder 2, **dadurch gekennzeichnet, dass** der Resonator (19) aus einem nichtmagnetischen Material besteht

4. Positioniervorrichtung nach Anspruch 1, 2 oder 3, **dadurch gekennzeichnet, dass** jede Feder zum Andrücken den an der Basis (2) abgestützten Resonators (19) an die Vorschubeinheit (3) aus Kunststoff ausgebildet ist.

5. Positionlenrorrichtung nach Anspruch 3, **dadurch gekennzeichnet, dass** jede Feder zum Andrücken des an der Basis (2) abgestützten Resonators (19) an die Vorschubeinheit (3) ein elastisches Band (11) ist, das parallel zu dem Resonator (19) verläuft und an dem Ende des Resonators (19), an dem dieser verschwenkbar an der Basis (2) gelagert ist, an dem Resonator (19) angreift, während es an dem Ende (12) das Resonators (19), an dem dieser an der Vorschubeinheit (3) angreift, an der Basis (2) abgestützt ist.

6. Positiontervorrichtung nach einem der Ansprüche 1 bis 5, **dadurch gekennzeichnet, dass** der Resonator (19) unter einem Winkel von 45 bis 55° zu der Vorschubeinheit ausgerichtet Ist.

7. Positioniervorrichtung nach einem der Ansprüche 1 bis 6, **dadurch gekennzeichnet, dass** der Resonator (19) an einer Schubstange (13) der Vorschubeinheit (3) angreift.

8. Positioniervorrichtung nach Anspruch 7, **dadurch gekennzeichnet, dass** die Schubstange (13) ein Rohrabschnitt (15) aus mit Kohlenstofffasern verstärktem Kunststoff ist.

9. Positioniervorrichtung nach Anspruch 7 oder 8, **dadurch gekennzeichnet, dass** die Schubstange (13) an der Basis (2) über Kugeln (17) aus keramischem Material geführt ist.

10. Positioniervorrichtung nach einem der Ansprüche 1 bis 9, **dadurch gekennzeichnet, dass** ein Hochfrequenzgenerator zur Erzeugung eines elektrischen Hochfrequenzsignals für den Wandler (20) von einer entfernten Steuereinheit aus über eine drahtlose Signalüberbtragungsstrecke ansteuerbar ist.

11. Positioniervorrichtung nach Anspruch 10, **dadurch gekennzeichnet, dass** Messsignale von der Mikroelektrode (16) über die drahtlose Signalobertragungsstrecke an die Steuereinheit übertragen werden.
